# EUROPEAN PATENT SPECIFICATION

(11) **EP 2 473 277 B1**
(45) Date of publication and mention of the grant of the patent: **03.07.2019**
(21) Application number: 10747645.9
(22) Date of filing: 01.09.2010
(51) Int. Cl.: B01J 31/02, B01J 31/24, C07C 51/00, C07C 51/14

(54) **PALLADIUM CATALYST SYSTEM COMPRISING ZWITTERION OR ACID-FUNCTIONALIZED IONIC LIQUID**
PALLADIUMKATALYSATORSYSTEM UMFASSEND ZWITTERION ODER SÄUREFUNKTIONALISIERTE IONISCHE FLÜSSIGKEIT
SYSTÈME CATALYTIQUE A BASE DE PALLADIUM COMPRENANT ZWITTERION OU LIQUIDE IONIQUE FONCTIONNALISÉ PAR DES ACIDES

(30) Priority: 03.09.2009 EP 09011313
(43) Date of publication of application: 11.07.2012
(73) Proprietor: Technical University of Denmark, 2800 Lyngby (DK)
(72) Inventor: GARCIA SUAREZ, Eduardo J, E-33004 Oviedo-Asturias (ES); RIISAGER, Anders, DK-2630 Taastrup (DK); FEHRMANN, Rasmus, DK-2100 Copenhagen Ø (DK); XIONG, Jianmin, DK-2830 Virum (DK)
(74) Representative: Zacco Denmark A/S
(86) International application number: PCT/EP2010/062797
(87) International publication number: WO 2011/026860

(56) References cited:
- WO-A1-96/19434
- WO-A1-2006/122563
- CN-A- 1 865 235
- COLE A C ET AL: "Novel Bronsted Acidic Ionic Liquids and Their Use as Dual Solvent-Catalysts", JOURNAL OF THE AMERICAN CHEMICAL SOCIETY, AMERICAN CHEMICAL SOCIETY, WASHINGTON, DC. US, vol. 124, 7 May 2002 (2002-05-07), pages 5962-5963, XP002447834, ISSN: 0002-7863
- LI, X. ET AL.: "Synthesis of multicarboxylic acid appended imidazolium ionic liquids and their application in palladium-catalyzed selective oxidation of styrene", NEW JOURNAL OF CHEMISTRY, vol. 31, 9 August 2007 (2007-08-09), pages 2088-2094, XP002556846,
- LI ET AL: "Synthesis of dioctyl phthalate using acid functionalized ionic liquid as catalyst", CATALYSIS COMMUNICATIONS, ELSEVIER SCIENCE, AMSTERDAM, NL, vol. 8, no. 11, 1 November 2007 (2007-11-01), pages 1759-1762, XP022262222, ISSN: 1566-7367
- DENIS SINOU ET AL: "Catalytic Asymmetric Alkylation in Water in the Presence of Surfactants", ADVANCED SYNTHESIS & CATALYSIS, vol. 345, no. 3, 1 March 2003 (2003-03-01) , pages 357-363, XP55339231, DE ISSN: 1615-4150, DOI: 10.1002/adsc.200390040

## Description

### Field of the invention

The present invention concerns a catalyst system comprising Palladium (Pd) and a zwitterion or an acid-functionalized ionic liquid, and one or more phosphine ligands, as defined in and by the appended claims. Such catalyst systems are suitable for e.g. carboxylation-, alkoxycarbonylation, and/or polymerization reactions, including Supported Ionic Liquid Phase (SILP) applications.

### Background of the invention

Methyl methacrylate (MMA) is an organic compound with the formula CH₂=C(CH₃)CO₂CH₃. This colourless liquid, the methyl ester of methacrylic acid (MAA) is a monomer produced on a large scale for the production of polymethyl methacrylate (PMMA), e.g. plexiglass

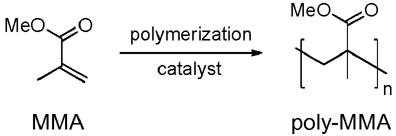

The principal application, consuming approximately 80% of the MMA, is the manufacture of polymethyl methacrylate acrylic plastics. Methyl methacrylate is also used for the production of the co-polymer methyl methacrylate-butadiene-styrene (MBS), used as a modifier for PVC.

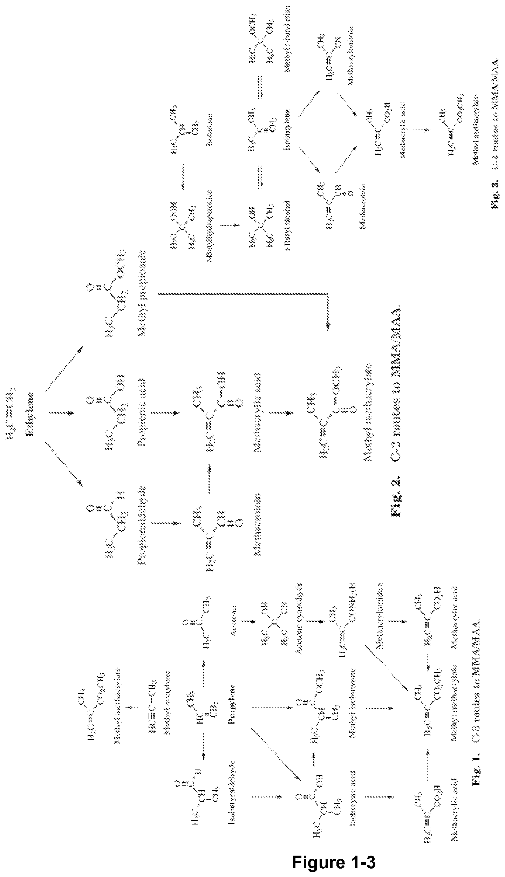

MMA can be manufactured by several methods, the principal one being the acetone cyanohydrin (ACH) route, using acetone and hydrogen cyanide as raw materials. The intermediate cyanohydrin is converted with sulfuric acid to a sulfate ester of the methacrylamide, methanolysis of which gives ammonium bisulfate and MMA. Although widely used, the ACH route coproduces substantial amounts of ammonium sulfate. Some producers start with an isobutylene or, equivalently, tert-butanol, which is sequentially oxidized first to methacrolein and then to methacrylic acid, which is then esterified with methanol. Propene can be carbonylated in the presence of acids to isobutyric acid, which undergoes subsequent dehydrogenation. Different synthesis routes are illustrated in **Figures 1-3**. The combined technologies afford more than 3 billion kilograms per year. MMA can also be prepared from methyl propionate and formaldehyde.

It is known that ethylene can be methoxycarbonylated to methylpropionate using a Pd catalyst:

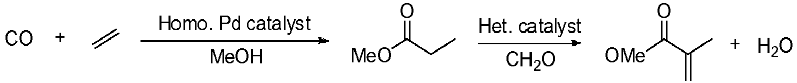

Wang et al. US 7 115 763 concerns Palladium catalyst systems for copolymerization reaction of alkenes, comprising "zwitterionic complexes". These zwitterionic complexes are different from the zwitterions and/or the acid functionalized ionic liquids according to the present invention.

Different Palladium catalyzed reactions, such as hydrogenations, Suzuki- and Heck coupling reactions etc. have been performed in ionic liquids, and WO06122563 pertains to a process for continuous carbonylation by supported ionic liquid-phase (SILP) catalysis.

Balázs et al. (2006) reported Palladium-catalyzed alkoxycarbonylation of styrene in conventional ionic liquids with phosphines and added Brønsted acids.

WO-A-96/19434 D1 discloses the carbonylation of ethylene using carbon monoxide and an alcohol, in the presence of a catalyst system comprising palladium and a phosphine ligand.

Surprisingly and unexpectedly, the inventors have found that stability and reusability of a Pd catalyst system can be improved by the use of one or more zwitterion(s) or one or more acid-functionalized ionic liquid(s) without inducing significant change of activity.

When using acid-functionalized ionic liquids a phase-separable system is obtainable after reaction, where the product and the ionic liquid catalyst can be retained in separate phases due to mutual low miscibility. This allows easy separation of the product and reuse of the ionic liquid catalyst phase.

Due to the non-volatile nature of acid-functionalized ionic liquids, the Pd catalyst systems according to the invention are also believed to be suitable for SILP catalysis.

### Summary of the invention

The present invention concerns a catalyst system comprising Palladium (Pd), a zwitterion or an acid-functionalized ionic liquid, wherein the Pd catalyst can be provided by a complex precursor, as defined in and by the appended claims. Such catalyst systems can be used for e.g. alkoxycarbonylation reactions, carboxylation reactions, and/or in a co-polymerization reaction. Catalyst systems according to the invention are suitable for reactions forming separable product and catalyst phases and supported ionic liquid phase SILP applications.

Thus, in a **first aspect**, the present invention concerns a catalyst system comprising a palladium (Pd) catalyst and a zwitterion, or an acid-functionalized ionic liquid, and one or more phosphine ligands, as defined in appended claim 1.

According to an embodiment, the catalyst system comprises a Pd catalyst, an acid-functionalized ionic liquid, and one or more phosphine ligands.

According to an embodiment, the system comprises a two-phase system, wherein the Pd catalyst and the reaction product are essentially contained in different phases.

According to an embodiment, the catalyst is a Supported Ionic Liquid-Phase (SILP) catalyst.

A **second aspect** of the invention relates to the use of a catalyst system according to the first aspect for catalyzing the reaction: R^{r}₁R^{r}₂C=CHR^{r}₃ + CO + R^{r}₄OH → R^{r}₁R^{r}₂CH-CHR^{r}₃COOR^{r}₄, as defined in appended claim 8.

According to an embodiment, the catalyst is used in the production of methyl methacrylate and/or in the production of methacrylic acid.

### Figures

**Figure 1** shows the C-3 routes to methyl methacrylate/methacrylic acid (MMA/MAA).
**Figure 2** shows the C-2 routes to MMA/MAA.
**Figure 3** shows the C-4 routes to MMA/MAA.

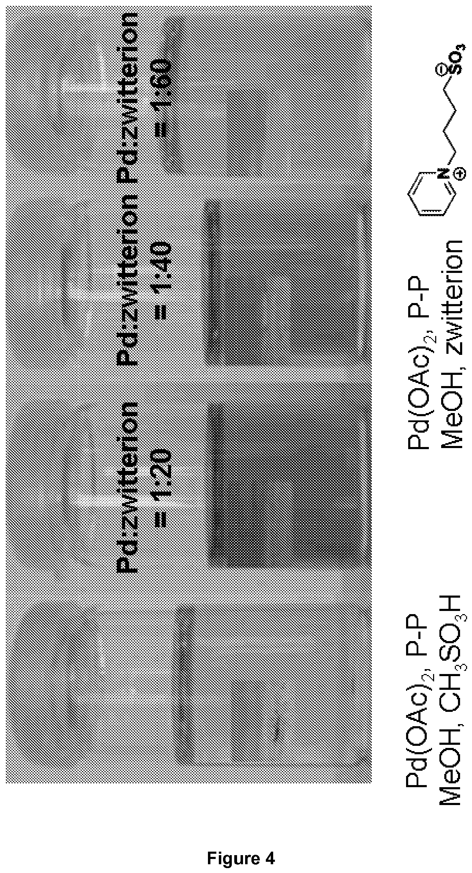

**Figure 4** shows Pd catalyst solutions with added acid and increasing concentrations of added zwitterion, respectively (left to right).

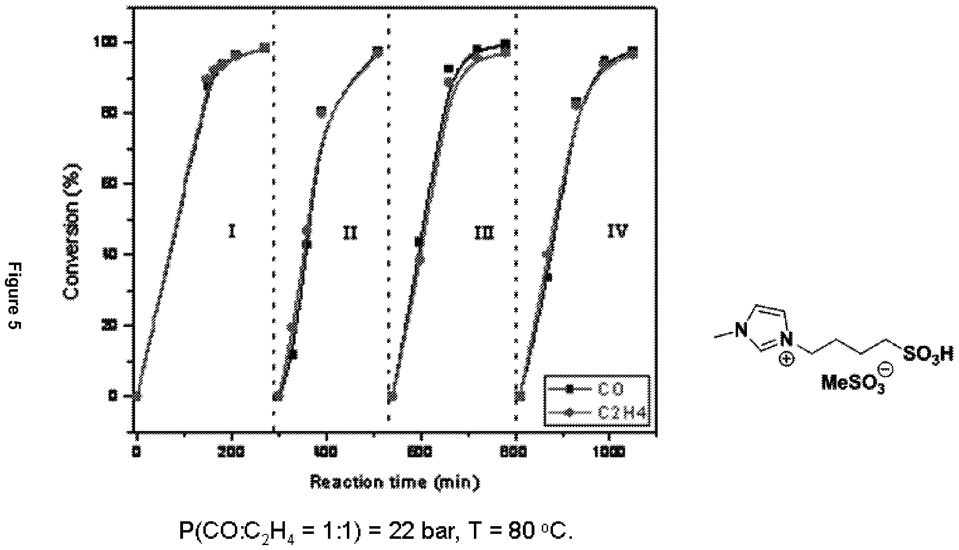

**Figure 5** shows the consecutive use of the acid-functionalized Pd catalyst system.
**Figure 6** shows a reaction mixture before (left) and after reaction (right), whereby a two-phase system is provided.
**Figure 7** shows different embodiments of phosphines.

### Detailed description of the invention

In the context of the present disclosure the terms "around", "about", "∼" or "approximately" are used interchangeably and refer to the claimed value, and may include variations as large as +/-0.1%, +/-1%, or +/-10%. Especially in the case of log₁₀ intervals, the variations may be larger and include the claimed value +/- 50%, or 100%. The terms "around", "about", "∼", or "approximately" may also reflect the degree of uncertainty and/or variation that is common and/or generally accepted in the art.

In the context of the present invention, the terms "optionally" and "alternatively" can be used interchangeably. Often, these terms are used to indicate optional and/or alternative embodiment(s).

### Definition of substituents

As used in the present invention, the term "C₁-C₂₀ alkyl" refers to a straight chained or branched saturated hydrocarbon having from one to twenty carbon atoms inclusive. Examples of such groups include, but are not limited to, methyl, 2-propyl, 1-butyl, 2-butyl, 2-methyl-2-propyl, 2-methyl-1-butyl, 1-hexyl, 1-octyl, 1-decyl and 1-dodecyl.

Similarly, the term "C₁-C₆ alkyl" refers to a straight chained or branched saturated hydrocarbon having from one to six carbon atoms inclusive.

Similarly, the term "C₁-C₄ alkyl" refers to a straight chained or branched saturated hydrocarbon having from one to four carbon atoms inclusive.

As used herein, the term "C₃-C₆ cycloalkyl" typically refers to cyclopropyl, cyclobutyl, cyclopentyl and cyclohexyl.

As used herein, the term "C₁-C₆ alkoxy" refers to a straight chain or branched saturated alkoxy group having from one to six carbon atoms inclusive with the open valency on the oxygen. Examples of such groups include, but are not limited to, methoxy, ethoxy, n-butoxy, 2-methyl-pentoxy and n-hexyloxy.

As used herein, the term "C₁-C₆ alkoxides" refers to the anions of straight chain or branched saturated alkanols having from one to six carbon atoms inclusive with the negative charge on the oxygen atom. Typical examples include methoxide and *tert*-butoxide.

As used herein, the term "alkene" refers to a straight chained, branched or cyclic hydrocarbon having from one to twenty carbon atoms inclusive, and one, two or more double bonds which may be isolated or conjugated.

As used herein, the term "halide" refers to the anion of a halogen atom.

As used herein, the term "phosphine" refers to compounds of the general formula (II) as defined below. Analogously, as used herein, the term "phosphine oxides" refers to phosphines of the general formula (II) which have been oxidized to their related oxides.

As used herein, the terms "arene" and "aryl" both refer to a mono- or bicyclic aromatic group having from six to twelve carbon atoms inclusive. Examples of such groups include, but are not limited to, phenyl, naphthyl, indenyl, tetrahydronaphthyl and indanyl.

As used herein, the term bi(C₆-C₁₂aryl) refers to two mono- or bicyclic aromatic groups joined by a single bond, each group having from six to twelve carbon atoms inclusive. A typical non-limitating example of such groups is biphenyl.

As used herein, the term aryl-C₁-C₄ alkyl refers to an aryl group as defined above, substituted with a C₁-C₄ alkyl as defined above, with the open valency on the terminal carbon of the C₁-C₄ alkyl group. Examples of such groups include, but are not limited to, benzyl and phenylethyl.

As used herein, the term "arsine" refers to compounds of formula As(R)₃ wherein R may be the same or different and selected from C₁-C₆ alkyl and aryl, as defined above.

As used herein, the term "heteroaryl" refers to a mono- or bicyclic heteroaromatic group having from five to ten carbon atoms and from one to three heteroatoms inclusive, wherein the heteroatoms are selected individually from N, O and S. Examples of such groups include, but are not limited to, pyridyl, 2-thienyl, 3-thienyl, 2-furyl, 3-furyl, quinolinyl and naphthyridyl.

### Abbreviations

Unless defined differently, the following abbreviations are meant to comprise the following:
Bn: Benzyl
COD: 1,5-Cyclooctadiene
Cy: Cyclohexyl
Cyp: Cyclopentyl
ICy: 1,3-Dicyclohexylimidazol-2-ylidene
IⁱPr: 1,3-Di-iso-propylimidazol-2-ylidene
IMe: 1,3-Dimethylimidazol-2-ylidene
IMes: 1,3-Dimesitylimidazol-2-ylidene
Imid: Imidazole
I^{t}Bu: 1,3-Di-tert-butylimidazol-2-ylidene
KHMDS: Potassium bis(trimethylsilyl)amide
Me: Methyl
Mes: Mesityl, i.e. 2,4,6-trimethylphenyl
NHC: N-Heterocyclic carbene
Q: Quantitative
RCM: Ring closing metathesis

### Catalyst

The metal catalyst according to the invention is or comprises Pd. Pd catalyst complexes are known to form a dark or black precipitate known as "Palladium black" upon decomposition. These are believed to consist of elemental palladium. The catalyst system can be provided by, formed by, or be derived from a complex precursor.

### Dissolved Pd complex

In the context or the present invention, the term "dissolved Pd complex" is meant to comprise a Pd complex formed by dissolution of one or more Pd precursor complex(es) in a solvent.

### Complex precursor(s)

In the context or the present invention, the term "complex precursor(s)" is meant to comprise a complex precursor(s) having formula **PdX^{c}ₘ^{c}**, **PdX^{c}ₘ^{c}Y^{c}ₙ^{c}**; **or PdZ^{c}ₒ^{c}**, wherein **X^{c}** can be selected independently from the group comprising or consisting of one or more of R^{c}₁R^{c}₂R^{c}₃CCOO, where R^{c}₁, R^{c}₂, and R^{c}₃ are independently selected from H, Cl, Br, I, or F; CH₃COO, CF₃COO, Cl, Br, I, NO₃, SO₄, acetylacetonate, dibenzylideneacetone, tricyclohexylphosphine, and tri-o-tolylphosphine, including any mixture(s) thereof; wherein **Y^{c}** can be selected independently from the group comprising or consisting of one or more of norbornadiene, 1,5-cyclooctadiene, tri-o-tolylphosphine, triphenylphosphine, tris-t-butylphosphine, tricyclohexylphosphine, 1,2-bis(diphenylphosphino)ethane, 1,3-bis(diphenylphosphino)-propane, benzonitrile, acetonitrile, 2-Bis(phenylsulfinyl)ethane, and 2-methylally, including any mixtures thereof; wherein **Z^{c}** can be selected independently from the group comprising or consisting of one or more of triphenylphosphine, acetonitrile, tris-t-butylphosphine, and dibenzylideneacetone, including any mixtures thereof; and wherein "**m^{c}**", "**n^{c}**", or "**o^{c}**" can be selected independently, e.g. from 1, 2, 3 and/or 4, such as m^{c} = 1 or 2, n^{c} = 1, and o^{c} = 4.

### Zwitterion

In the context or the present dislosure, the term "zwitterion" is meant to comprise an overall neutrally charged chemical molecule which carries formal positive and negative charges on different atoms in the molecule. Such a zwitterion can e.g. be a covalent molecule of formula Q^{z}-R^{z}-S^{z}, wherein **Q^{z}** is one or more positively charged functional groups, such as functional groups selected from the group comprising or consisting of quaternary nitrogen-, quaternary phosphorous-, ternary sulphur-, ternary selene, optionally substituted with one or more organic group(s) including optionally substituted, linear or branched-chained C₁-C₂₀ alkyl group(s), optionally substituted C₆-C₁₈ aryl group(s) and/or optionally substituted cyclic group(s) with 4-12 carbon atoms, wherein the ring of the cyclic group comprises one or more heteroatoms, such as nitrogen, oxygen and/or sulphur, including any combination thereof; wherein **R^{z}** is an organic bridging group or spacer e.g. selected from the group comprising or consisting of one or more of 1-20 covalently bound carbon atoms including optionally substituted, linear or branched-chained C₁-C₁₀ alkylidene group, for example, trimethylene and tetramethylene, or optionally substituted C₆-C₁₈ aryl group(s) or optionally substituted cyclic group(s) with 4-12 carbon atoms, wherein the ring of the cyclic group comprises one or more heteroatoms, such as nitrogen, oxygen, sulphur, including any combination thereof, and exemplary substituents on the organic bridging group comprise e.g. halogens, alkoxy and carboxy groups; and wherein **S^{z}** is one or more negatively charged functional groups, such as functional groups selected from the group comprising or consisting of carboxylate, thiocarboxylate, carbonate, sulfonate, sulfate, selenonate, selenate, phosphonate and phosphate, optionally including substituted, linear or branched-chained C₁-C₂₀ alkyl, optionally substituted with one or more C₆-C₁₈ aryl, or optionally substituted cyclic or N-, O-, or S-heterocyclic hydrocarbyl derivatives thereof. Concerning **Q^{z}**, **R^{z}**, and/or **S^{z}**, all groups can be selected independently, and suitable alkyl groups include, among others, methyl, ethyl, propyl, isopropyl, tert-butyl, cyclopentyl, cylohexyl or cyclooctyl. Exemplary cyclic groups containing heteroatoms include, among others, 1-pyridyl and 3-methylimidazyl. Aryl groups include, for example, phenyl, benzyl, cumenyl, mesityl, tolyl and xylyl.

### Zwitterion complex

In the context or the present disclosure, the term "zwitterion complex" is meant to comprise a neutrally charged entity consisting of or comprising a positively charged central metal ion coordinated to one or more negatively charged ligand via electron pair donation to the central metal ion.

### Phosphine

In the context of the present disclosure, the term "phosphine" is usually meant to comprise a monodentate phosphine of formula I: **(R^{p}₁)₃-P**, or a bidentate phospine of formula II:

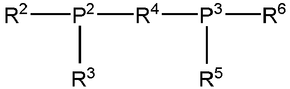

In formula I **P** represent a phosphorus atom, and **R^{p}₁** represent an optionally substituted organic group. This organic group is preferable a C₁-C₂₀ alkyl group, a C₆-C₁₈ aryl group or a cyclic group with 4-12 carbon atoms in which the ring of the cyclic group also contains one or more heteroatoms, such as nitrogen. Alkyl groups include, among others, methyl, ethyl, propyl, isopropyl, tert-butyl, cyclopentyl, cylohexyl or cyclooctyl. Exemplary cyclic groups containing heteroatoms include, among others, 6-methyl-2-pyridyl and 4,6-dimethyl-2-pyridyl. Aryl groups include, for example, naphthyl, phenyl, benzyl, cumenyl, mesityl, tolyl and xylyl. The organic group can be substituted, for example, with halogen atoms, for example Cl, Br or F, or with C₁-C₆ alkyl, C6-C18 aryl, C1-C6 alkoxy, carboxy, carbalkoxy, acyl, trihalogenmethyl, cyano, dialkylamino, sulphonylalkyl or alkanoyloxy groups. Substituents can be groups with electron withdrawing or electron donating properties.

Monodentate phosphine ligands include, for instance, triphenylphosphine, trip-tolylphophine, tri-o-tolylphophine, dimethylphenyl-phosphine, ethyldiphenyl phosphine or cyclohexyldiphenylphosphine.

In phosphine formula II, **P²** and **P³** represent phosphorus atoms, and **R^{p}₂**, **R^{p}₃**, **R^{p}₅**, **and R**^{p}₆ represent optionally substituted organic group(s), which they can be the same or different. R^{p}₂, R^{p}₃, R^{p}₅, and R^{p}₆ can be any group as specified earlier for R^{p}₁. R^{p}₄ is an organic bridging group having 3-20 carbon atoms, optionally R^{p}₄ can be any group as defined for **R^{z}**.

Furthermore, two groups selected from any one of R^{p}₂, R^{p}₃, R^{p}₅, and R^{p}₆, while bonded to one P-atom can form a divalent organic group, for example a diaryl group or a C₂-C₂₀ alkylene group. An exemplary alkenyl group is butenyl. Examples of diaryl groups include diphenyl and dinaphthyl groups.

The substituents for the organic groups R^{p}₂, R^{p}₃, R^{p}₅, and R^{p}₆ can be the same as described above for the monodentate phosphine ligands. Examples of possible aliphatic and aryl groups can comprise the groups described for R^{p}₂, R^{p}₃, R^{p}₅, and R^{p}₆.

Divalent organic bridging groups, such as R^{p}₄ can consist or comprise C4-C10 alkylidene groups, for example tetramethylene, pentamethylene or trans-1,2-cyclobutene; and C6-C20 divalent arylgroups such as, for example, dinaphythyl or diphenyl. One hetero atom may be present in R^{p}₄, for example nitrogen, oxygen or sulphur.

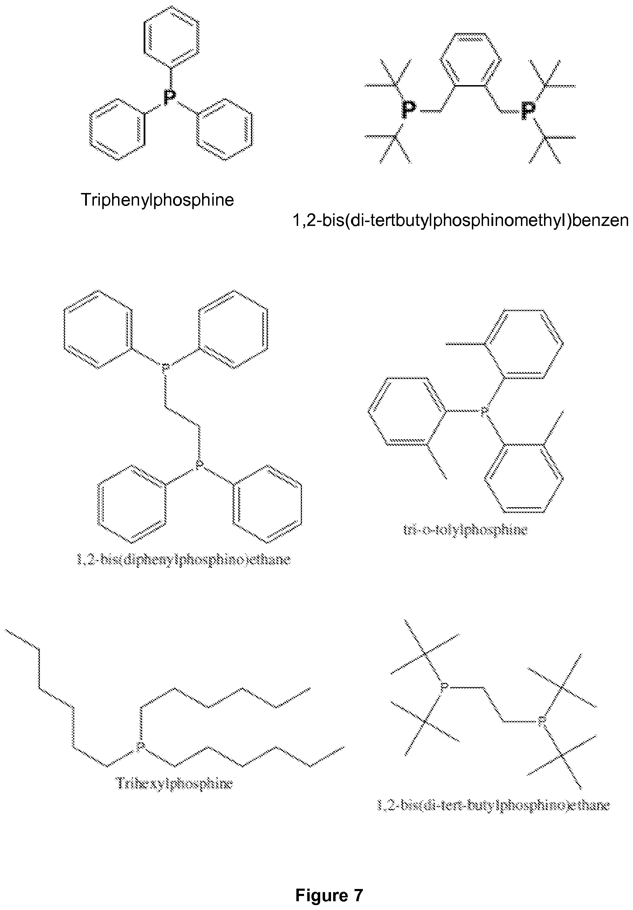

Suitable phosphine ligands in the context of the present invention comprise e.g. one or more phosphines shown in **Figure 7**, such as triphenylphosphine, trihexylphospine, tricyclohexylphosphine, (PCy₃), 1,2-bis(di-tertbutyl-phosphino-methyl)benzene ("(P-P)" or "(P-P) Ligand"), tri-o-tolylphosphine, and 1,2-bis(di-tert-butylphosphino)ethane.

### Acid

In the context of the present disclosure, the term "acid" is meant to comprise one or more inorganic and/or organic Brønsted acid(s), including any combination thereof. A Brønsted-Lowry acid (or simply Brønsted acid) is a species that donates a proton to a Brønsted-Lowry base.

### Volatile - non-volatile

In the context of the present disclosure, the terms volatile and/or non-volatile are usually used in connection with one or more compounds. Generally, non-volatile compounds are characterized by having a non-measurable, negligible, and/or insignificant vapor pressure at conditions where the compound is intended to be used. On the contrary, a volatile compound has a measurable, non-negligible and/or significant vapor pressure and may evaporate under its use.

### Acid-functionalized ionic liquid

In the context of the present invention, the term "acid-functionalized ionic liquid" is meant to comprise a combination of one or more cation(s) **A** and one or more anion(s) **B**, of formula **[AH]⁺[B]**⁻. "H" indicates a Bronsted acid proton with a pKa of usually less than 5. Said Brønsted acid proton H may e.g. originate from a carboxylic acid, sulfonic acid or phosphonic acid group covalently comprised in said A and/or B cations; wherein A can be selected from the group comprising or consisting of one or more of quaternary nitrogen-, quaternary phosphorous-, ternary sulphur-, and ternary selen-based cation(s), including any combination thereof; and B can be selected from the group comprising or consisting of one or more of halide(s), nitrate(s), sulphate(s), sulfonate(s), sulfonyl amide(s), phosphate(s), borate(s), antimonite(s), acetate(s), including optionally any hydrogen-, halogen-, hydroxyl-, or C₁-C₆ alkoxy-substituted hydrocarbyl derivative(s) thereof. Acid-functionalized ionic liquid may comprise compounds, wherein the cation A is a carboxylic acid and/or, sulfonic acid and/or phosphonic acid and/or any derivative thereof, optionally selected from the group comprising or consisting of (i), (ii), (iii), (iv), or (v); wherein
(i) has formula: 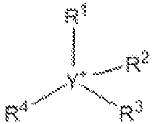 wherein R^{f}₁, R^{f}₂, R^{f}₃, and R^{f}₄ are independently selectable from the group consisting of optionally substituted, linear or branched-chained C₁-C₂₀ alkyl, optionally substituted cyclic C₃-C₂₀ alkyl, and optionally substituted C₆-C₂₀ aryl; and Y is N or P;
(ii) has formula: 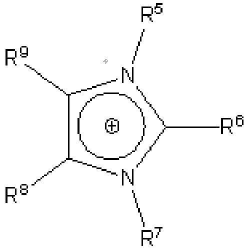
   wherein R^{f}₅ and R^{f}₇ are independently selected from the group consisting of optionally substituted, linear or branched-chained C₁-C₂₀ alkyl, optionally substituted cyclic C₃-C₂₀ alkyl, and optionally substituted C₆-C₂₀ aryl; and
   R^{f}₆, R^{f}₈ and R^{f}₉ are independently selected from hydrogen, optionally substituted, linear or branched-chained C₁-C₂₀ alkyl, optionally substituted cyclic C₃-C₂₀ alkyl, and optionally substituted C₆-C₂₀ aryl;
(iii) has formula: 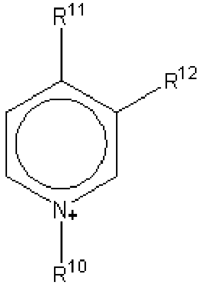
   wherein R^{f}₁₀ is independently selectable from the group consisting of optionally substituted, linear or branched-chained C₁-C₂₀ alkyl, optionally substituted cyclic C₃-C₂₀ alkyl, and optionally substituted C₆-C₂₀ aryl; and
   R^{f}₁₁ and R^{f}₁₂ are independently selectable from hydrogen, optionally substituted, linear or branched-chained C₁-C₂₀ alkyl, optionally substituted cyclic C₃-C₂₀ alkyl, and optionally substituted C₆-C₂₀ aryl;
(iv) has formula: 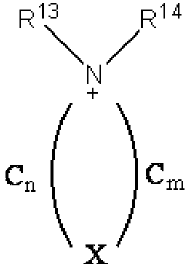 wherein R^{f}₁₃ and R^{f}₁₄ are independently selectable from the group consisting of optionally substituted, linear or branched-chained C₁-C₂₀ alkyl, optionally substituted cyclic C₃-C₂₀ alkyl, and optionally substituted C₆-C₂₀ aryl; X is C, N, O, or S; n^{f} and m^{f} are independently from each other integers from 0 to 6 with the proviso that the sum 1≤ m^{f}+n^{f} ≤ 6; and
(v) has formula: 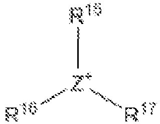 wherein R^{f}₁₅, R^{f}₁₆, and R^{f}₁₇ are independently selectable from the group consisting of optionally substituted, linear or branched-chained C₁-C₂₀ alkyl, optionally substituted cyclic C₃-C₂₀ alkyl, and optionally substituted C₆-C₂₀ aryl; and Z is S or Se.

### Solvent

In the context of the present invention, the term "solvent" is meant to comprise a reaction media, compound and/or composition, wherein a solid or liquid compound can be dissolved forming a homogeneous solution. The solvent is usually in a liquid state of matter before, during or after use and/or reaction according to the invention. The solvent can be a mixture of liquids including substrate(s) and/or reaction products like water, on or more ionic liquids and/or one or more organic liquids, such as e.g. alcohol(s). The solvent can e.g. be liquid at room or environmental temperature, or under reaction conditions. An ionic liquid and/or an acid-functionalized ionic liquid can also be a solvent.

### Supported Ionic Liquid-Phase (SILP)

In the context of the present invention, the terms "Supported Ionic Liquid-Phase" or "SILP" can be used interchangeably and are meant to comprise e.g. any example or embodiment as disclosed in WO06122563, Often, a SILP catalyst is supported on a porous support. Suitable support material(s) may comprise e.g. silicas, organic polymers, carbon, zeolites, clays, alumina, titania, zirconia, and any mixture(s) and combination(s) thereof.

A **first aspect** of the present invention concerns a catalyst system comprising a palladium (Pd) catalyst and a zwitterion, or an acid-functionalized ionic liquid, and one or more phosphine ligands, as defined by the appended claims.

Thus, according to an embodiment, the catalyst system comprises a Pd catalyst, one or more zwitterion(s), and one or more phosphine ligands. According to another embodiment, the catalyst system comprises a Pd catalyst, one or more acid-functionalized ionic liquid(s), and one or more phosphine ligands.

According to a further embodiment, the catalyst system comprises Pd catalyst, one or more zwitterion(s), one or more acid-functionalized ionic liquid(s), and one or more phosphine ligands.

According to an embodiment, the system comprises a two-phase system, wherein the Pd catalyst and the reaction product are essentially contained in different phases.

According to an embodiment, the catalyst is a Supported Ionic Liquid-Phase (SILP) catalyst.

According to an embodiment, the catalyst system comprises a dissolved Pd complex. Said complex can e.g. be provided from or formed via one or more complex precursor(s). Often, but not exclusively, the complex precursor will comprise or consist of one or more palladium (II) or palladium (0) complex precursor(s). Suitable complex precursor(s) may comprise e.g. one or more of is Pd(CH₃COO)₂, PdCl₂, Pd(CH₃COCHCOCH₃), Pd(CF₃COO)₂, Pd(PPh₃)₄ or Pd₂(dibenzylideneacetone)₃, including any derivative(s), combination(s) and/or mixture(s) thereof.

In case of a catalyst system comprising a zwitterion, or one or more zwitterion(s), suitable zwitterion(s) is(are) selected from the group comprising or consisting of 1-(4-sulfonylbutyl)pyridinium, 1-(4-sulfonylbutyl)3-methylimidazolium, 1-(4-sulfonylbutyl)triethylammonium, and 1-(4-sulfonylbutyl)tri-phenylphosphonium, including any combination(s) and/or mixture(s) thereof. According to an embodiment of the invention, the one or more zwitterion(s) can be a covalent molecule of formula Q^{z}-R^{z}-S^{z} as defined above.

A catalyst system according to the invention also comprises one or more phosphine(s). According to an embodiment the one or more phosphine(s) is a covalent molecule of
formula I: (R^{p}₁)₃-P, or a bidentate phospine of formula II: as defined above.

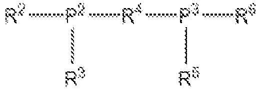

According to an embodiment, the one or more phosphine ligands can be selected from the group comprising or consisting of monophosphine(s), biphosphine(s), monodentate phosphine(s), triphenylphosphine, trihexylphosphine, tricyclohexylphosphine, tri-o-tolylphosphine, bidentate phosphine(s), 1,2-bis(di-tert-butylphosphino)ethane, 1,2-bis(diphenyl-phosphino)ethane, and 1,2-bis((di-tert-butylphosphino)methyl)benzene, and any combination and/or mixtures thereof. According to a further embodiment, the one or more phosphine(s) is a phosphine illustrated in Figure 7.

A catalyst system according to the invention may also comprise one or more acids, such as one or more inorganic and/or organic Brønsted acid(s). Suitable acids in the context of the present invention are meant to comprise e.g. any acid(s) is/are selected from the group comprising or consisting of one or more of methanesulfonic acid, ethanesulfonic acid, 2-butanesulfonic acid, propanesulfonic acid, 1,2-ethanedisulfonic acid, benzenesulfonic acid, ethyl-benzenesulfonic acid, fluorosulfonic acid, chlorosulfonic acid, sulfamic acid, perfluorooctane sulfonic acid, octanesulfonic acid, 1-naphthalenesulfonic acid, 2-naphthalenesulfonic acid, 1,5-naphthalenedisulfonic acid, methionic acid, o-cresolsulfonic acid, phenol-4-sulfonic acid, 2-pyridinesulfonic acid, 4-iodobenzenesulfonic acid, 4-chlorobenzenesulfonic acid, p-toluenesulfonic acid, 4-biphenylsulfonic acid, phenylhydrazine-4-sulfonic acid, phenol-4-sulfonic acid, 1,3-propanedisulfonic acid, 1,4-butanedisulfonic acid, dimethyl-benzenesulfonic acid, 5-sulfosalicylic acid dehydrate, formic acid, oxalic acid, 2,6-dipicolinic acid, tricarballylic acid, sulfuric acid, hydrochloric acid, hydrobromic acid, hydroiodic acid, nitric acid, perchloric acid, trifluoromethylsulfonic acid, and fluorosulfonic acid-pentafluoroantimon magic acid; and any combination(s) and mixture(s) thereof. According to a further embodiment, the phosphine is a phosphine illustrated in Figure 7.

According to an embodiment of the invention, the catalyst system comprises one or more zwitterion(s), and one or more acid(s).

According to an embodiment of the invention, the catalyst system comprises one or more solvents.

According to an embodiment of the invention, the catalyst system comprises one or more ionic liquid(s), wherein said ionic liquid is a salt composed entirely or essentially of ions, wherein said salt is melted at reaction conditions, and the one or more ionic liquid is acid-functionalized ionic liquid(s).

According to an embodiment of the invention, the catalyst system comprises one or more solvents, and one or more ionic liquid(s).

According to another embodiment, the acid-functionalized ionic liquid and/or ionic liquid acts as solvent

According to an embodiment of the invention, the catalyst system comprises (a) a Pd catalyst; (b) one or more acid-functionalized ionic liquid(s) and (c) one or more phosphine ligands. According to another embodiment, the catalyst system does not comprise any further acid apart from the functionalized ionic liquid(s).

According to an embodiment of the invention, the acid-functionalized ionic liquid comprises a combination of one or more cation(s) A and one or more anion(s) B, the acid-functionalized ionic liquid having formula [AH]⁺[B]⁻ as defined above, wherein the "H" of the acid-functionalized ionic liquid is a Bronsted acid proton, i.e. a proton originating from a Bronsted acid, wherein the Bronsted acid has a pKa of around 5, or less than 5, or alternatively around 4, or less than4.

According to an embodiment of the invention, the one or more acid-functionalized ionic liquid(s) can be selected from the group comprising or consisting of 1-(4-sulfonylbutyl)pyridinium hydrogensulfate, 1-(4-sulfonylbutyl)triethylammonium hydrogensulfate, 1-(4-sulfonylbutyl)-imidazolium hydrogensulfate, 1-(4-sulfonylbutyl)imidazolium methanesulfonate and 1-(4-carboxylbutyl)imidazolium chloride, including any combination thereof.

According to the invention, suitable acid-functionalized ionic liquid of type [AH]⁺[B]⁻ may also comprise any compound(s) selected from the group comprising or consisting of one or more of 1-methyl-3-(4-sulfobutyl)imidazolium trifluoromethylsulfonate, 1-methyl-3-(4-sulfobutyl)imidazolium p-toluenesulfonate, 1-methyl-3-(4-sulfobutyl)imidazolium methanesulfonate, 1-methyl-3-(4-sulfobutyl)imidazolium hydrogensulfate, 1-methyl-3-(4-sulfobutyl)imidazolium trifluoroacetate, 1-methyl-3-(4-sulfobutyl)imidazolium chloride, 1-methyl-3-(4-sulfobutyl)imidazolium bromide, 1-methyl-3-(4-sulfobutyl)imidazolium nitrate, 1-methyl-3-(4-sulfobutyl)imidazolium tetrafluoroborate, 1-methyl-3-(4-sulfobutyl)imidazolium hexafluorophosphate, 1-methyl-3-(4-sulfobutyl)imidazolium diethylphosphate, 1-methyl-3-(4-sulfobutyl)imidazolium bis(trifluoromethylsulfonyl)amide, 1-methyl-3-(3-sulfopropyl)imidazolium trifluoromethylsulfonate, 1-methyl-3-(3-sulfopropyl)imidazolium methanesulfonate, 1-ethyl-3-(4-sulfobutyl)imidazolium trifluoromethylsulfonate, 1-ethyl-3-(4-sulfobutyl)imidazolium p-toluenesulfonate, 1-ethyl-3-(4-sulfobutyl)imidazolium methanesulfonate, 1-ethyl-3-(4-sulfobutyl)imidazolium hydrogensulfate, 1-ethyl-3-(4-sulfobutyl)imidazolium trifluoroacetate, 1-ethyl-3-(4-sulfobutyl)imidazolium chloride, 1-ethyl-3-(3-sulfopropyl)imidazolium trifluoromethylsulfonate, 1-ethyl-3-(3-sulfopropyl)imidazolium methanesulfonate, 1-butyl-3-(4-sulfobutyl)imidazolium trifluoroacetate, 1-butyl-3-(4-sulfobutyl)imidazolium trifluoromethylsulfonate, 1-butyl-3-(4-sulfobutyl)imidazolium p-toluenesulfonate, 1-butyl-3-(4-sulfobutyl)imidazolium methanesulfonate, 1-butyl-3-(4-sulfobutyl)imidazolium hydrogensulfate, 1-butyl-3-(3-sulfopropyl)imidazolium trifluoromethylsulfonate, 1-butyl-3-(3-sulfopropyl)imidazolium methanesulfonate, triphenyl(4-sulfobutyl)phosphonium trifluoroacetate, triphenyl(4-sulfobutyl)phosphonium trifluoromethylsulfonate, triphenyl(4-sulfobutyl)phosphonium p-toluenesulfonate, triphenyl(4-sulfobutyl)phosphonium methanesulfonate, triphenyl(4-sulfobutyl)phosphonium hydrogensulfate, triphenyl(3-sulfopropyl)phosphonium trifluoromethylsulfonate, triphenyl(3-sulfopropyl)phosphonium methanesulfonate, 1-(4-sulfobutyl)pyridinium trifluoroacetate, 1-(4-sulfobutyl)pyridinium trifluoromethylsulfonate, 1-(4-sulfobutyl)pyridinium p-toluenesulfonate, 1-(4-sulfobutyl)pyridinium methanesulfonate, 1-(4-sulfobutyl)pyridinium hydrogensulfate, 1-(4-sulfobutyl)pyridinium chloride, 1-(3-sulfopropyl)pyridinium trifluoromethylsulfonate, 1-(3-sulfopropyl)pyridinium methanesulfonate, 1-methyl-3-(4-sulfobutyl)pyrrolidinium trifluoroacetate, 1-methyl-3-(4-sulfobutyl)pyrrolidinium trifluoromethylsulfonate, 1-methyl-3-(4-sulfobutyl)pyrrolidinium p-toluenesulfonate, 1-methyl-3-(4-sulfobutyl)pyrrolidinium methanesulfonate, 1-methyl-3-(4-sulfobutyl)pyrrolidinium hydrogensulfate, 1-methyl-3-(3-sulfopropyl)pyrrolidinium trifluoromethylsulfonate, 1-methyl-3-(3-sulfopropyl)pyrrolidinium methanesulfonate, N-(4-sulfobutyl)trimethylammonium trifluoroacetate, N-(4-sulfobutyl)trimethylammonium trifluoromethylsulfonate, N-(4-sulfobutyl)trimethylammonium p-toluenesulfonate, N-(4-sulfobutyl)trimethylammonium methanesulfonate, N-(4-sulfobutyl)trimethylammonium hydrogensulfate, N-(3-sulfopropyl)trimethylammonium trifluoroacetate, N-(3-sulfopropyl)trimethylammonium methanesulfonate, N-(4-sulfobutyl)triethylammonium trifluoroacetate, N-(4-sulfobutyl)triethylammonium trifluoromethylsulfonate, N-(4-sulfobutyl)triethylammonium p-toluenesulfonate, N-(4-sulfobutyl)triethylammonium methanesulfonate, N-(4-sulfobutyl)triethylammonium hydrogensulfate, N-(3-sulfopropyl)triethylammonium trifluoromethylsulfonate, N-(3-sulfopropyl)triethylammonium methanesulfonate, N-(4-sulfobutyl)tributylammonium trifluoroacetate, N-(4-sulfobutyl)tributylammonium trifluoromethylsulfonate, N-(4-sulfobutyl)tributylammonium p-toluenesulfonate, N-(4-sulfobutyl)tributylammonium methanesulfonate, N-(4-sulfobutyl)tributylammonium hydrogensulfate, N-(3-sulfopropyl)tributylammonium methanesulfonate, N-(3-sulfopropyl)-tributylammonium trifluoromethylsulfonate, dimethyl(4-sulfobutyl)sulfonium trifluoromethylsulfonate, dimethyl(4-sulfobutyl)sulfonium methanesulfonate, diethyl(4-sulfobutyl)sulfonium trifluoromethylsulfonate, diethyl(4-sulfobutyl)-sulfonium methanesulfonate, and any mixture(s) and combination(s) thereof.

According to an embodiment, through not falling within the scope of the invention, suitable acid-functionalized ionic liquid of type [A]⁺[BH]⁻ may comprise any compound(s) selected from the group comprising or consisting of one or more of 1-methyl-3-methylimidazolium hydrogensulfate, 1-ethyl-3-methylimidazolium hydrogensulfate, 1-ethyl-3-ethylimidazolium hydrogensulfate, 1-ethyl-3-ethylimidazolium hydrogenselenate, 1-butyl-3-methylimidazolium hydrogensulfate, 1-butyl-3-methylimidazolium hydrogenselenate, 1-hexyl-3-methylimidazolium hydrogensulfate, 1-methyl-3-octylimidazolium hydrogensulfate, N-methylpyridinium hydrogensulfate, N-butylpyridinium hydrogensulfate, N-butylpyridinium hydrogenselenate, N-dimethylpyrrolidinium hydrogensulfate, 1-butyl-1-methylpyrrolidinium hydrogensulfate, trihexyl(tetradecyl)phosphonium hydrogensulfate, tetramethylammonium hydrogensulfate, tetramethylammonium hydrogenselenate, tetraethylammonium hydrogensulfate, tetraethylammonium hydrogenselenate, tetrabutylammonium hydrogensulfate, tetrabutylammonium hydrogenselenate, and triethylsulfonium hydrogensulfate, and any mixture(s) and combination(s) thereof.

According to an embodiment the catalyst system comprises a Pd catalyst, a zwitterion and one or more phosphine ligands, but said system does not comprise acid, ionic liquid, acid-functionalized ionic liquid, and zwitterion complex. According to another embodiment, the catalyst system comprises a Pd catalyst and an acid-functionalized ionic liquid and one or more phosphine ligands, but said system does not comprise acid, ionic liquid other than the acid-functionalized ionic liquid, zwitterion, and zwitterion complex. According to a further embodiment, the catalyst system comprises a Pd catalyst, a zwitterion and an acid-functionalized ionic liquid and one or more phosphine ligands, but said system does not comprise acid, ionic liquid other than the acid-functionalized ionic liquid, zwitterion, and zwitterion complex. According to an embodiment of the invention, the catalyst system comprises a two-phase system, and wherein the Pd catalyst and the reaction product are essentially contained in different phases.

According to the invention, the catalyst system can be suitable for a Supported Ionic Liquid-Phase (SILP) application. According to an embodiment, the catalyst is a Supported Ionic Liquid-Phase (SILP) catalyst. The SILP catalyst can be confined in the ionic liquid catalyst solution on a porous support, and the porous support can e.g. be selected from the group comprising or consisting of silica(s), organic polymer(s), carbon(s), zeolite(s), clay(s), alumina(s), titania(s), zirconia(s), and any derivative(s), mixture(s) and combination(s) thereof. According to an embodiment, the catalytically active Pd is present in an amount of up to 25% by weight, preferably up to 10% by weight. According to an embodiment of the invention, the SILP catalyst comprises Pd present in an amount of up to 5, 10, 15, 20 or 25% by weight. In another embodiment, the SILP catalyst comprises Pd present in an amount of up to around 10% by weight. According to an embodiment, the (acid functionalized) ionic liquid is present in an amount of e.g. up to 66%, up to 50%, or up to 33% by weight,

According to an embodiment the catalyst system catalyzes the reaction:

R^{r}₁R^{r}₂C=CHR^{r}₃ + CO + R^{r}₄OH → R^{r}₁R^{r}₂CH-CHR^{r}₃COOR^{r}₄,

or

(R^{r}₁R^{r}₂C=CHR^{r}₃)ₙ^{r} + m^{r}CO → (-R^{r}₁R^{r}₂C-CHR^{r}₃-CO-)ₙ^{r} + (m^{r}-n^{r})CO

wherein R^{r}₁, R^{r}₂, R^{r}₃, and R^{r}₄ can be selected independently from the group consisting of H, CH₃, CH₃CH₂, alkyl, aryl, cyclic group, C₁-C₂₀ alkyl group, C₆-C1₈ aryl group, cyclic group with 4-12 carbon atoms, optionally comprising one or more heteroatoms, such as nitrogen in the ring of the cyclic group; n^{r} = 2-10.000 (or 100.000) or more; and m^{r} is greater than n^{r}. In another embodiment, R^{r}₁, R^{r}₂, and R^{r}₃ are H. In a further embodiment, R^{r}₁, R^{r}₂, and R^{r}₃ are H, and R^{r}₄ is CH₃.

An aspect of the disclosure relates to reactions catalyzed by a catalyst system according to the first aspect, comprising e.g. carboxylation reactions, alkoxycarbonylation reactions and/or polymerization reactions.

According to an embodiment, the reactions are e.g.:

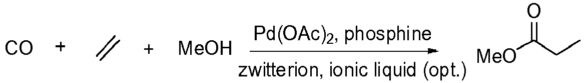

R^{r}₁R^{r}₂C=CHR^{r}₃ + CO + R^{r}₄OH → R^{r}₁R^{r}₂CH-CHR^{r}₃COOR^{r}₄,

and/or

ₙ^{r}(R^{r}₁R^{r}₂C=CHR^{r}₃) + m^{r}CO → (-R^{r}₁R^{r}₂C-CHR^{r}₃-CO-)ₙ^{r} + (m^{r}-n^{r})CO;

wherein R^{r}₁, R^{r}₂, R^{r}₃, and R^{r}₄ can be selected and/or defined as described above.

In an embodiment of the invention, the substrate is an ethylenically unsaturated compound, such as an olefin or a mixture of olefins. Suitable ethylenically unsaturated compounds include, among others, ethylene, propylene, butylene, isobutylene, pentene, pentene nitriles, methyl 3-pentenoates, 2-and 3-pentenoic acid.

A second **aspect** of the invention pertains to the use of a catalyst or catalyst system according to the first aspect, e.g. in a reaction as defined in appended claim 8.

According to an embodiment, the catalyst is used in the production of methyl methacrylate and/or in the production of methacrylic acid.

According to an embodiment a catalyst system according to the first aspect is used in an alkoxycarbonylation reaction, a carbonylation reaction, a carboxylation reaction, and/or in a polymerization reaction.

According to another embodiment of the invention, the catalyst system is used in an alkoxycarbonylation reaction, a carboxylation reaction, and/or in a co-polymerization reaction, optionally in the production of methyl propionate and/or propanoic acid, optionally in processes forming methyl methacrylate and/or methacrylic acid.

According to a further embodiment of the invention, the catalyst system is used in the production of methyl methacrylate and/or in the production of methacrylic acid and/or in the production of poly-MMA.

An aspect of the disclosure concerns a method of providing a catalyst according to the first aspect.

It is also shown that the stability and re-use of palladium catalyst complexes can be increased in the presence of zwitterions and/or ionic liquids in alkoxycarbonylation, where an alkene, CO and an alcohol are selectivity reacted to produce esters.

Use and/or application of zwitterion(s) and/or acid-functionalized ionic liquid(s) as co-catalysts and/or stabilizing agents and/or processing aid(s) make it possible to efficiently perform palladium catalyzed alkoxycarbonylation without acid additives, thus simplifying processing.

Without wanted to be bound by any theory, it is believed that more expensive phosphine(s) can be replaced by less expensive phosphine(s), providing e.g. a cost reduction.

Application of ionic liquids as solvent provide a phase-separable reaction system that e.g. allow easy separation of the product (e.g. by decantation) and reuse of the palladium catalyst system e.g. through effective immobilization.

Without wanting to be construed as limiting to the present invention, experimental evidence is presented in the following section. This comprises examples of the palladium catalyst system with zwitterions/ionic liquids. Catalyzed reactions comprise methoxycarbonylation of ethylene with CO and methanol to yield methylpropionate.

Without wanting to be limited by any theory, it is believed that acid-functionalized ionic liquids or ionic liquids are practically non-volatile, and often, they possess a low flammability and/or toxicity. As solvent, they have a wide liquid range, good thermal/chemical stability and tunable solubility properties. They improve stability of metal catalyst, and their viscosity may limit diffusion in bulk systems. Roles for acid-functionalized ionic liquids or ionic liquids may comprise their use as catalyst, co-catalyst, ligand source, reaction media, and separation media

The invention is further exemplified in the following, non-limiting examples, including Tables and Figures.

### EXAMPLES

### Example 1

### 1-(4-sulfonylbutyl)pyridinium

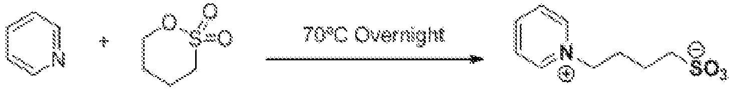

A mixture of pyridine (0.077 mol) and 1,4-butanesultone (0.07 mol) were heated with stirring at 70°C overnight. On completion, the obtained white solid was washed three times with 80 mL of diethylether. Finally, the product was dried under vaccum. **Yield 97.6%.**

### Example 2

### 1-(4-sulfonylbutyl)3-methylimidazolium

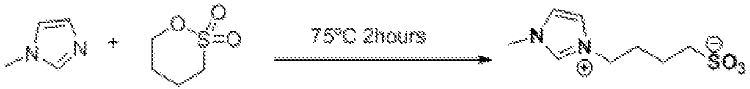

A mixture of 3-methylimidazole (0.099 mol) and 1,4-butanesultone (0.09 mol) were heated with stirring at 75°C for 2 hours. On completion, the obtained white solid was washed three times with 80 mL of diethylether. Finally, the product was dried under vaccum. **Yield 93.4%.**

### Example 3

### 1-(4-sulfonylbutyl)tri-ethylamonium

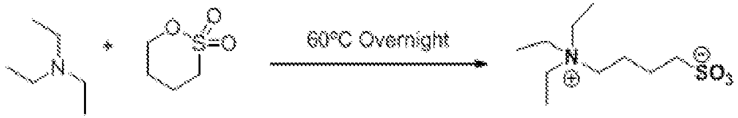

A mixture of triethylamine (0.077 mol) and 1,4-butanesultone (0.07 mol) were heated with stirring at 60°C overnight. On completion, the obtained white solid was washed three times with 80 mL of diethylether. Finally, the product was dried under vacuum. **Yield 60.5%.**

### Example 4

### 1-(4-sulfonylbutyl)tri-phenylphosphonium

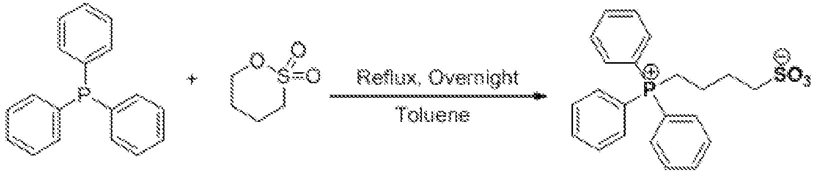

A mixture of triphenylphosphine (0.05 mol) and 1,4-butanesultone (0.045 mol) in 30 mL of toluene were refluxed (aprox. 120°C) with stirring overnight. On completion, the obtained white solid was washed two times with 20 mL of toluene and three times with 80 mL of diethylether. Finally, the product was dried under vacuum. **Yield 7.2%.**

Without wanting to be bound by any theory, the low yield could be explained by the volume of toluene used and the reaction conditions chosen. This zwitterion showed very poor solubility in MeOH.

### Example 5

### 1-(4-sulfonylbutyl)pyridinium hydrogensulfate

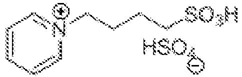

A mixture of 1-(4-sulfonylbutyl)pyridinium (0.077 mol) and sulfuric acid (0.07 mol) were heated with stirring at 60°C overnight. During this time the solid liquefy, resulting in the formation of 1-(4-sulfonylbutyl)pyridinium hydrogensulfate as a slighty yellow sticky oil that was dried under vacuum overnight.
**Yield 97.6%.**

### Example 6

### 1-(4-sulfonylbutyl)triethylammonium hydrogensulfate,

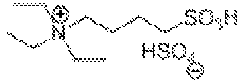

A mixture of 1-(4-sulfonylbutyl)triethylammonium (0.077 mol) and sulfuric acid (0.07 mol) were heated with stirring at 60°C overnight. During this time the solid liquefy, resulting in the formation of 1-(4-sulfonylbutyl)triethylammonium hydrogensulfate as a slighty yellow sticky oil that was dried under vacuum overnight. **Yield 92.7%.**

### Example 7

### 1-(4-carboxylbutyl)imidazolium chloride

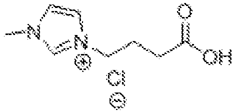

A mixture of 1-methylimidazole (0.077 mol) and 4-chlorobutanoic acid (0.05 mol) were heated with stirring at 70°C overnight. On completion ether was added. The solid-oil was washed with ether (50mLx3). The slighty yellow sticky oil was dried under vacuum overnight.

### Example 8

### 1-(4-sulfonylbutyl)imidazolium hydrogensulfate,

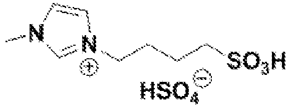

A mixture of 1-(4-sulfonylbutyl)imidazol (0.077 mol) and sulfuric acid (0.07 mol) were heated with stirring at 60°C overnight. During this time the solid liquefy, resulting in the formation of 1-(4-sulfonylbutyl)imidazoilum hydrogensulfate as a slighty yellow sticky oil that was dried under vacuum overnight.

### Example 9

### 1-(4-sulfonylbutyl)imidazolium methanesulfonate

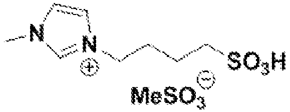

A mixture of 1-(4-sulfonylbutyl)imidazol (0.077 mol) and methanesulfonic acid (0.07 mol) were heated with stirring at 60°C overnight. During this time the solid liquefy, resulting in the formation of 1-(4-sulfonylbutyl)imidazoilum methanesulfonate as a slighty yellow sticky oil that was dried under vacuum overnight.

### Example 10 (not according to the invention)

### Catalytic Runs: (A) System without pre-activation

Catalytic reactions were carried out in a 50 mL Parr autoclave. The reactor containing palladium acetate (11.2 mg, 0.05 mmol), either no zwitterion or zwitterion (1, 2, 3, or 4 = zwitterion from Example 1, 2, 3, or 4) 1 mmol zwitterion (Pd/Zwitterion = 1/20), 2 mmol (Pd/Zwitterion = 1/40) or 3 mmol zwitterion (Pd/Zwitterion = 1/60), 1,2-bis((di-*tert*-butylphosphino) methyl)benzene (0.25 mmol) dissolved in methanol was purged with a gas mixture CO/ethylene/N₂ (40/40/20) 3 times. The autoclave was then pressurised to 20 bar using the same gas mixture CO/ethylene/N₂ (40/40/20) and heated from R.T. to 80 °C. The pressure was allowed to fall during the reaction period. After the reaction time, the reaction was terminated by rapid cooling of the autoclave and the unreacted gases vented. The reaction slurry was then filtered and the liquid kept for GC analysis. The activity for methyl propionate formation was determined by gas chromatography.

Selected results from the experiments are comprised in Table I.

### Example 11 (not according to the invention)

### Catalytic Runs: (B) Re-use of the catalytic system

After terminating the reaction by rapid cooling of the autoclave, the unreacted gases were vented. Then for run 2, the autoclave was charged again with the gas mixture (20bar) and heated from R.T. (room temperature) to 80°C. The pressure was allowed to fall during the reaction period. After the reaction time, the reaction was terminated by cooling the autoclave and the unreacted gases were vented. The same procedure was followed for the re-use of the catalytic system.

Selected results from the experiments are comprised in Table II.

### Example 12 (not according to the invention)

### Catalytic Runs: (C) System with pre-activation using 1,2-bis((di-tert-butylphosphino)methyl or triphenyliphosphine as phosphine ligands

Catalytic reactions were carried out in a 50 mL Parr autoclave. The reactor containing palladium acetate (11,2 mg, 0.05 mmol), either no zwitterion or zwitterion (1, 2, 3, or 4 = zwitterion from Example 1, 2, 3, or 4) 1 mmol zwitterion (Pd/Zwitterion = 1/20), 2 mmol (Pd/Zwitterion = 1/40) or 3 mmol zwitterion (Pd/Zwitterion = 1/60), 1,2-bis((di-*tert*-butylphosphino)methyl)benzene (0.25 mmol) or triphenylphosphine (0.5 mmol (1:10) (Pd/P) or 1,25 mmol (1:25) (Pd/P)) dissolved in methanol was purged with Argon three times. The autoclave was then pressurised to 2 bar with Argon and heated at 80 °C overnight (12h, 14h or 16h). The autoclave was then cooled down to R.T. and purged with a gas mixture CO/ethylene/N₂ (40/40/20) 3 times. After that, the autoclave was pressurised to 20 bar using the same gas mixture CO/ethylene/N₂ (40/40/20) and heated from R.T. to 80 °C in the case of the 1,2-bis((di-*tert*-butylphosphino)methyl or to 100°C in the case of triphenylphosphine. The pressure was allowed to fall during the reaction period (batch mode). After the reaction time, the reaction was terminated by rapid cooling of the autoclave and the unreacted gases vented. The reaction slurry was then filtered and the liquid kept for GC analysis. The activity for methyl propionate was determined by gas chromatography.

The triphenylphosphonium zwitterion was only poorly soluble in MeOH. Without wanting to be bound by any theory, it is believed that this zwitterion could be more active in e.g. an alkoxycarbonylation of e.g. ethylene, once the appropriate solvent has been selected for solubilisation of the zwitterion.

Selected results from the experiments are comprised in Table III.

### Example 13

### Catalytic Runs: (D) System with acid-functionalized ionic liquids using 1,2-bis((di-tert-butylphosphino)methyl ligand

Catalytic reactions were carried out in a 50 mL Parr autoclave. The reactor containing palladium acetate (22.6 mg, 0.1 mmol) and 1,2-bis((di-*tert-*butylphosphino)methyl)benzene (0.5 mmol) (1:10) (Pd/P) dissolved in methanol alone or methanol-ionic liquid (1-butyl-3-methylimidazolium acetate or 1-butyl-3-methylimidazolium hydrogensulfate) with methanesulfonic acid or methanol with acid-functionalized ionic liquids from Examples 8 and 9, was purged with a gas mixture CO/ethylene/N₂ (40/40/20) three times. After that, the autoclave was pressurised to 20 bar using the same gas mixture CO/ethylene/N₂ (40/40/20) and heated from R.T. to 80 °C. The pressure was allowed to fall during the reaction period (batch mode). After the reaction time, the reaction was terminated by rapid cooling of the autoclave and the unreacted gases vented. The reaction slurry was then filtered and the liquid kept for GC analysis. The activity for methyl propionate was determined by gas chromatography.

Selected results from the experiments are comprised in Table IV, the second and third entries being examples according to the invention.

### Example 14

### Phase-separation: (E) System with acid-functionalized ionic liquids using 1,2-bis((di-tert-butylphosphino)methyl ligand

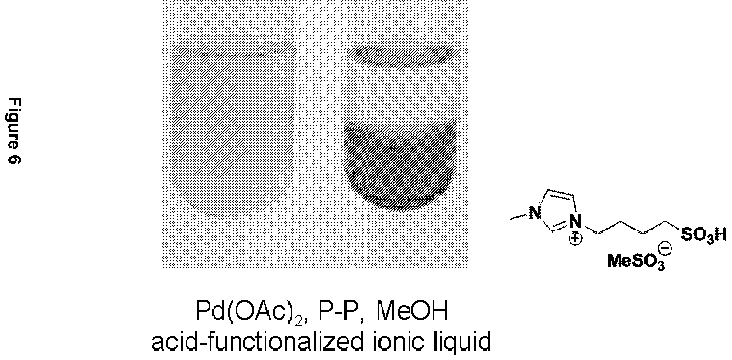

The reaction mixture with the acid-functionalized ionic liquid 1-(4-sulfonyl-butyl)imidazolium methanesulfonate with dissolved Pd catalyst, as described in Example 13, is shown in Figure 6. The homogeneous reaction mixture before reaction is shown to the left, and the phase separated reaction mixture after completion of the reaction to the right. The separated upper clear phase consists of the methyl propionate product with traces of methanol, while the lower coloured phase consists of the ionic liquid containing the dissolved Pd catalyst.

The example illustrates the facile separation of the product from the recyclable Pd catalyst system.

### TABLES

**Table I**

| **Methoxycarbonylation of ethylene with CO and MeOH ^{a}** | | | | |
|---|---|---|---|---|
| **Zwitterion** | **ReactionTime (hours)** | **Conversion (%)** | **Ratio (Pd/Zwitterion)** | **Product^{b}** |
| **No** | 8 | 42.1 | No | methylpropionate |
| **1** | 8 | 92.3 | (1/60) | methylpropionate |
| **2** | 8 | 84.6 | (1/60) | methylpropionate |
| **3** | 8 | 44.3 | (1/60) | methylpropionate |
| **4** | 8 | 72.7 | (1/40) | methylpropionate |
| **^{a}Pd(OAc)₂ 0,05 mmol; Ligand (P-P) 0.25 mmol; MeOH volume 6 mL; Total pressure (N2/CO/Ethylene) (1/2/2) 20 bar; Reaction temperature 80°C. ^{b}100% Selectivity** | | | | |

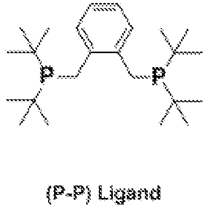

**Table II**

| **Methoxycarbonylation of ethylene with CO and MeOH^{a}** | | | | |
|---|---|---|---|---|
| **Zwitterion** | **ReactionTime (hours)** | **Conversion (%)** | **Ratio (Pd/Zwitterion)** | **Product^{b}** |
| **No-Run1** | 7 | 35.7 | No | methylpropionate |
| **No-Run2** | 7 | 17.1 | No | methylpropionate |
| **1-Run1** | 8 | 92.3 | (1/60) | methylpropionate |
| **1-Run2** | 1 | 90.1 | (1/60) | methylpropionate |
| **2-Run1** | 8 | 84.6 | (1/60) | methylpropionate |
| **2-Run2** | 1 | 91.9 | (1/60) | methylpropionate |
| **2-Run3** | 1 | 85.3 | (1/60) | methylpropionate |
| **3-Run1** | 8 | 44.3 | (1/60) | methylpropionate |
| **3-Run2** | 8 | 22.3 | (1/60) | methylpropionate |
| **4-Run1** | 8 | 72.7 | (1/60) | methylpropionate |
| **4-Run2** | 6 | 11.5 | (1/60) | methylpropionate |
| **^{a}Pd(OAc)₂ 0.05 mmol; Ligand (P-P) 0.25 mmol; MeOH volume 6 mL; Total pressure (N2/CO/Ethylene) (1/2/2) 20 bar; Reaction temperature 80°C; ^{b}100% Selectivity** | | | | |

**Table III**

| **Methoxycarbonylation of ethylele with CO and MeOH^{a} (pre-activation mode)** | | | | | |
|---|---|---|---|---|---|
| **Zwitterion** | **ReactionTime (hours)** | **Conversion (%)** | **Ratio (Pd/Ligand/Zwitterion)** | **Pre-activation Time (hours)** | **Product^{c}** |
| **No** | 6 | 0 | (1/10/No) | 16 | methylpropionate |
| **1** | 5 | 89.3 | (1/25/60) | 14 | methylpropionate |
| **1** | 3 | 92.6 | (1/10/60) | 14 | methylpropionate |
| **2** | 1 | 93.8 | (1/10/60) | 16 | methylpropionate |
| **^{a}Pd(OAc)₂ 0.05 mmol; Ligand Triphenylphosphine; MeOH volume 6 mL; Total pressure (N2/CO/Ethylene) (1/2/2) 20 bar; Reaction temperature 100°C; ^{b}Pre-activation temperature 80°C;^{c} 100% Selectivity** | | | | | |

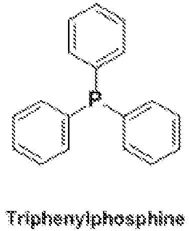

**Table IV: Acid-functionalized ionic liquids**

| **Acid promoters** | **Acidity (mmol H⁺/g)** | **TOF^{a} (h⁻¹)** | **Conversion (%)^{b}** | | **Linear selectivity (%)** |
|---|---|---|---|---|---|
| | | | **CO** | **C₂H₄** | |
| methanesulfonic acid | 10.3 | 193 | 99.2 | 98.7 | > 99 |
| 1-(4-sulfonylbutyl)imidazolium methanesulfonate | 3.17 | 97 | 93.7 | 93.5 | > 99 |
| 1-(4-sulfonylbutyl)imidazolium hydrogensulfate, | 6.57 | 96 | 87.1 | 86.1 | > 99 |
| 1-butyl-3-methylimidazolium acetate | 0.06 | 0 | -- | -- | -- |
| 1-butyl-3-methylimidazolium hydrogensulfate | 4.21 | 0 | -- | -- | -- |

| | | | | | |
|---|---|---|---|---|---|
| MeOH volume 4 mL. ^{a} Turnover frequency [mol CO consumed per mol Pd and h] after 60 min calculated according to the gas-uptake curves. ^{b} Data after 3 hours reaction. P(CO:C₂H₄ = 1:1) = 22 bar, T = 80 °C. | | | | | |

## Claims

1. A catalyst system for alkoxycarbonylation reactions comprising a palladium (Pd) catalyst and one of a) or b):
a) a zwitterion selected from the group consisting of 1-(4-sulfonylbutyl)pyridinium, 1-(4-sulfonylbutyl)3-methylimidazolium, 1-(4-sulfonylbutyl)triethylammonium, and 1-(4-sulfonylbutyl)tri-phenylphosphonium and one or more phosphine ligands selected from the group of triphenylphosphine, trihexylphosphine, tricyclohexylphosphine, tri-o-tolylphosphine, 1,2-bis(di-tert-butylphosphino)-ethane, 1,2-bis(diphenylphosphino)ethane, and 1,2-bis((di-tert- butylphosphino)methyl)-benzene, and any combination(s) and/or mixture(s) thereof and one or more inorganic and/or organic Bronsted acid(s);
b) an acid-functionalized ionic liquid comprising a combination of one or more cation(s) A and one or more anion(s) B, the acid-functionalized ionic liquid having formula [AH]⁺[B]⁻, wherein the "H" is a proton originating from a Bronsted acid with a pKa of less than 5 and one or more phosphine ligands selected from the group of trihexylphosphine, tricyclohexylphosphine, tri-o-tolylphosphine, 1,2-bis(di-tert-butylphosphino)ethane, 1,2-bis(diphenylphosphino)ethane, and 1 ,2-bis((ditert- butylphosphino)-methyl)benzene, and any combination(s) and/or mixture(s) thereof.

2. A catalyst system according to claim 1, wherein the Pd catalyst comprises a dissolved Pd complex.

3. A catalyst system according to claim 2, wherein the dissolved Pd complex is provided or formed from one or more complex precursor(s).

4. A catalyst system according to claim 2, wherein the dissolved Pd complex is provided or formed from one or more palladium (II) or palladium (0) complex precursor(s).

5. A catalyst system according to claim 4, wherein said complex precursor(s) is Pd(CH₃COO)₂, PdCl2, Pd(CH₃COCHCOCH₃), Pd(CF₃COO)₂, Pd(PPh₃)₄ or Pd₂(dibenzylideneacetone)₃.

6. A catalyst system according to any of the preceding claims, wherein the acid-functionalized ionic liquid is selected from the group consisting of 1-(4-sulfonylbutyl)pyridinium hydrogensulfate, 1-(4-sulfonylbutyl)triethylammonium hydrogensulfate, 1-(4-sulfonylbutyl)imidazolium hydrogensulfate, 1-(4-sulfonylbutyl)imidazolium methanesulfonate and 1-(4-carboxylbutyl)-imidazolium chloride, including any combination thereof.

7. A catalyst system according to any of the preceding claims, wherein the catalyst is a Supported Ionic Liquid-Phase (SILP) catalyst.

8. Use of a catalyst system according to any one of claims 1-7 for catalyzing the reaction:
R^{r}₁R^{r}₂C=CHR^{r}₃ + CO + R^{r}₄OH → R^{r}₁R^{r}₂CH-CHR^{r}₃COOR^{r}₄
wherein R^{r}₁, R^{r}₂, R^{r}₃, and R^{r}₄ can be selected independently from the group consisting of H, CH₃, CH₃CH₂, C₁-C₂₀ alkyl group, C₆-C₁₈ aryl group or a cyclic group with 4-12 carbon atoms.

## Patentansprüche

1. Katalysatorsystem für Alkoxycarbonylierungsreaktionen umfassend einen Palladium (Pd)-Katalysator und eines aus a) oder b):
a) ein Zwitterion ausgewählt aus der Gruppe bestehend aus 1-(4-Sulfonylbutyl)pyridinium, 1-(4-Sulfonylbutyl)3-methylimidazolium, 1-(4-Sulfonylbutyl)triethylammonium und 1-(4-Sulfonylbutyl)triphenylphosphonium und einen oder mehrere Posphinligand(en) ausgewählt aus der Gruppe bestehend aus Triphenylphosphin, Trihexylphosphin, Tricyclohexylphosphin, Tri-o-tolylphosphin, 1,2-Bis(di-tert-butylphosphino)-ethan, 1,2-Bis(diphenyl-phosphino)ethan und 1,2-Bis((di-tert-butylphosphino)methyl)-benzen, und irgendeine Kombination(en) und/oder Mischung(en) davon und eine oder mehrere anorganische(n) und/oder organische(n) Brønsted-Säure(n);
b) eine säurefunktionalisierte ionische Flüssigkeit umfassend eine Kombination aus einem oder mehreren Kation(en) A und einem oder mehreren Anion(en) B, wobei die säurefunktionalisierte ionische Flüssigkeit die Formel [AH]⁺[B]⁻ aufweist, wobei das "H" ein Proton ist, das von einer Brønsted-Säure mit einem pKa von weniger als 5 stammt, und ein oder mehrere Phosphinligand(en) ausgewählt aus der Gruppe aus Trihexylphosphin, Tricyclohexylphosphin, Tri-o-tolylphosphin, 1,2-Bis(di-tert-butylphosphino)-ethan, 1,2-Bis(diphenylphosphino)ethan und 1,2bis((ditert-butylphosphino)-methyl)benzen, und irgendeine Kombination(en) und/oder Mischung(en) davon.

2. Katalysatorsystem nach Anspruch 1, wobei der Pd-Katalysator einen gelösten Pd-Komplex umfasst.

3. Katalysatorsystem nach Anspruch 2, wobei der gelöste Pd-Komplex aus einem oder mehreren Komplexvorläufer(n) bereitgestellt oder gebildet ist.

4. Katalysatorsystem nach Anspruch 2, wobei der gelöste Pd-Komplex aus einem oder mehreren Palladium (II)- oder Palladium (0)-Komplexvorläufer(n) bereitgestellt oder gebildet ist.

5. Katalysatorsystem nach Anspruch 4, wobei der bzw. die Komplexvorläufer Pd(CH₃COO)₂, PdCl2, Pd(CH₃COCHCOCH₃), Pd(CF₃COO)₂, Pd(PPh₃)₄ oder Pd₂(Dibenzylidenacetone)₃ ist bzw. sind.

6. Katalysatorsystem nach einem der vorgehenden Ansprüche, wobei die säurefunktionalisierte ionische Flüssigkeit aus der Gruppe bestehend aus 1-(4-Sulfonylbutyl)pyridiniumhydrogensulfat, 1-(4-Sulfonylbutyl)triethylammonium-hydrogensulfat, 1-(4-Sulfonylbutyl)imidazoliumhydrogensulfat, 1-(4-Sulfonyl-butyl)imidazoliummethansulfonat und 1-(4-Carboxylbutyl)imidazoliumchlorid umfassend irgendeine Kombination davon ausgewählt ist bzw. sind.

7. Katalysatorsystem nach einem der vorgehenden Ansprüche, wobei der Katalysator ein Supported lonic Liquid-Phase (SILP)-Katalysator ist.

8. Verwendung eines Katalysatorsystems nach einem der Ansprüche 1-7 zum Katalysieren der folgenden Reaktion:
R^{r}₁R^{r}₂C=CHR^{r}₃ + CO + R^{r}₄OH → R^{r}₁R^{r}₂CH-CHR^{r}₃COOR^{r}₄
wobei R^{r}₁, R^{r}₂, R^{r}₃ und R^{r}₄ unabhängig von der Gruppe bestehend aus H, CH₃, CH₃CH₂, C₁-C₂₀-Alkylgruppe, C₆-C₁₈-Arylgruppe oder einer zyklischen Gruppe mit 4-12 Kohlenstoffatomen ausgewählt werden können.

## Revendications

1. Système de catalyseur pour des réactions d'alcoxycarbonylation comprenant un catalyseur au palladium (Pd) et l'un d'a) ou de b) :
a) un zwitterion choisi dans le groupe constitué par 1-(4-sulfonylbutyl) pyridinium, 1-(4-sulfonylbutyl)3-méthylimidazolium, 1-(4-sulfonylbutyl) triéthylammonium et 1-(4-sulfonylbutyl) triphénylphosphonium et un ou plusieurs ligands phosphine choisis dans le groupe constitué par triphénylphosphine, trihexylphosphine, tricyclohexylphosphine, tri-o-tolylphosphine, 1,2-bis (di-tert-butylphosphino) éthane, 1,2-bis (diphénylphosphino) éthane et 1,2-bis ((di-tert-butylphosphino) méthyl) benzène et toute combinaison et / ou tout mélange de ceux-ci et un ou plusieurs acides de Brønsted inorganiques et / ou organiques ;
b) un liquide ionique à fonctionnalité acide comprenant une combinaison d'un ou plusieurs cations A et d'un ou de plusieurs anions B, le liquide ionique à fonctionnalité acide ayant la formule [AH]⁺[B]⁻, dans laquelle "H" est un proton provenant d'un acide de Brønsted avec un pKa inférieur à 5 et un ou plusieurs ligands phosphine choisis dans le groupe constitué par trihexylphosphine, tricyclohexylphosphine, tri-o-tolylphosphine, 1,2-bis (di-tert-butylphosphino) éthane, 1,2-bis (diphénylphosphino) éthane, et 1,2-bis ((di-tert-butylphosphino) méthyl) benzène et toute combinaison et / ou toute mélange de ceux-ci.

2. Système de catalyseur selon la revendication 1, dans lequel le catalyseur au Pd comprend un complexe de Pd dissous.

3. Système de catalyseur selon la revendication 2, dans lequel le complexe de Pd dissous est pourvu ou formé à partir d'un ou plusieurs précurseurs de complexe.

4. Système de catalyseur selon la revendication 2, dans lequel le complexe de Pd dissous est pourvu ou formé à partir d'un ou plusieurs précurseurs de complexe de palladium (II) ou de palladium (0).

5. Système de catalyseur selon la revendication 4, dans lequel le ou les précurseurs de complexe sont Pd(CH₃COO)₂, PdCl2, Pd(CH₃COCHCOCH₃), Pd(CF₃COO)₂, Pd(PPh₃)₄ ou Pd₂(dibenzylidèneacétone)₃.

6. Système de catalyseur selon l'une quelconque des revendications précédentes, dans lequel le liquide ionique à fonctionnalité acide est choisi dans le groupe constitué par 1-(4-sulfonylbutyl) pyridinium hydrogénosulfate, 1-(4-sulfonylbutyl) triéthylammonium hydrogénosulfate, 1-(4-sulfonylbutyl) imidazolium hydrogènesulfate, 1-(4-sulfonyl-butyl) imidazolium méthanesulfonate et 1-(4-carboxylbutyl) imidazolium chlorure, y compris toute combinaison de ceux-ci.

7. Système de catalyseur selon l'une quelconque des revendications précédentes, dans lequel le catalyseur est un catalyseur à phase liquide ionique supporté (SILP).

8. Utilisation d'un système de catalyseur selon l'une quelconque des revendications 1 à 7 pour catalyser la réaction :
R^{r}₁R^{r}₂C=CHR^{r}₃ + CO + R^{r}₄OH → R^{r}₁R^{r}₂CH-CHR^{r}₃COOR^{r}₄
dans laquelle R^{r}₁, R^{r}₂, R^{r}₃, et R^{r}₄ peuvent être choisis indépendamment dans le groupe constitué de H, CH₃, CH₃CH₂, groupe alkyle en C₁-C₂₀, groupe aryle en C₆-C₁₈ ou groupe cyclique avec 4 à 12 atomes de carbone.
